# EUROPEAN PATENT SPECIFICATION

(11) **EP 2 886 093 B1**
(45) Date of publication and mention of the grant of the patent: **21.09.2016**
(21) Application number: 13198446.0
(22) Date of filing: 19.12.2013
(51) Int. Cl.: A61F 13/49, A61F 13/532, A61F 13/534, A61F 13/537, A61F 13/539, A61F 13/42

(54) **Absorbent Article comprising one or more colored areas**
Saugfähiger Artikel mit einem oder mehreren gefärbten Bereichen
Article absorbant comprenant une ou plusieurs zones colorées

(43) Date of publication of application: 24.06.2015
(73) Proprietor: The Procter & Gamble Company, Cincinnati, OH 45202 (US)
(72) Inventor: Kreuzer, Carsten Heinrich, 53881 Euskirchen (DE); Ehrnsperger, Bruno Johannes, 65812 Bad Soden (DE); Bianchi, Ernesto Gabriel, 61440 Oberursel (DE)
(74) Representative: L'Huillier, Florent Charles

(56) References cited:
- EP-A1- 2 679 209
- WO-A1-2009/134626
- WO-A1-2012/014436
- WO-A1-2012/170778

## Description

### FIELD OF THE INVENTION

The invention is for an absorbent article for personal hygiene such as, but not limited to, baby diapers, training pants or adult incontinence products, and an absorbent core for use in such absorbent articles.

### BACKGROUND OF THE INVENTION

Absorbent articles for personal hygiene, such as disposable diapers for infants, training pants for toddlers or adult incontinence products, are designed to absorb and contain liquid bodily exudates, in particular large quantity of urine. These absorbent articles comprise several layers providing different functions, for example a topsheet, a backsheet and in-between an absorbent core, among other layers.

Absorbent articles comprising a wetness indicator are known. The wetness indicator can be a wetness indicator composition that indicates the presence of liquid bodily exudates in the absorbent article. The wetness indicator composition can comprise a colorant adapted to change in appearance, i.e. appear, disappear, change color, upon contact with liquid bodily exudates such as urine, runny bowel movements, or menses in the absorbent article.

Wetness indicators composition may typically comprise a colorant, a stabilizer and a matrix. Absorbent articles comprising a wetness indicator composition and an absorbent core which may be cellulose free have been disclosed for example in WO2010/120705 (Klofta).

Although the wetness indicator composition effectively adheres and remains where it is initially located, a premature color change may occur depending on the nature of the component of the absorbent articles being in close proximity to the wetness indicator composition. For example, when the absorbent core comprises increased levels of superabsorbent polymer and adhesives, the composition of these components may induce a premature change of color of the wetness indicator composition prior to any significant absorption of liquid bodily exudates into the absorbent article.

Also, other criteria need to be considered. For instance, it may be desirable that the color change of the wetness indicator composition occurs as quickly as possible or the color signaling any significant absorption of liquid bodily exudates remains visible for a long time.

Recently, new absorbent cores comprising one or more channels have been developed. For example, WO2012/170778 (Rosati et al.) discloses absorbent structures that comprise superabsorbent polymer, optionally a cellulosic material, and at least a pair of substantially longitudinally extending channels. The core wrap can be adhesively bonded through the channels to form a channel bond. As the absorbent structure absorbs liquid and swells, the absorbent structure takes a three-dimensional shape with the channels becoming visible.

There is a desire to implement a visual indicating system to inform the caregiver that the absorbent article has been partially or fully loaded with liquid bodily exudates. The visual indicating system should be easy to comprehend and shall not need to use a special wetness indicator composition.

### SUMMARY OF THE INVENTION

The invention is for an absorbent article for personal hygiene and an absorbent core for use in such absorbent article, as defined in the claims. An absorbent article 20 for personal hygiene comprises a liquid permeable topsheet 24, a liquid impermeable backsheet 25, and an absorbent core 28 between the topsheet 24 and backsheet 25. The absorbent core 28 comprises a core wrap enclosing an absorbent material 60. The absorbent material 60 comprises a superabsorbent polymer. The core wrap comprises a top side 16 facing the topsheet 24 and a bottom side 16' facing the backsheet 25. The absorbent core 28 comprises one or more substantially absorbent material free area(s) 26 through which a portion of the top side 16 of the core wrap is attached by one or more core wrap bond(s) 27 to a portion of the bottom side 16' of the core wrap. The top side 16 of the core wrap is at least colored in the portion of the top side 16 of the core wrap which is attached by one or more core wrap bond(s) 27 to the portion of the bottom side 16' of the core wrap so that the color of the top side 16 of the core wrap forms one or more colored area(s) 126 visible from the exterior of the absorbent article 20. When the absorbent material 60 swells, one or more core wrap bond(s) 27 at least partially open(s) so that the colored area(s) 126 becomes no longer or less visible from the exterior of the absorbent article 20.

This visual indicating system can help to provide an easy comprehension if the absorbent article has been partially or fully loaded with liquid bodily exudates. Also, this visual indicating system does not also need to use a special wetness indicator composition, as it will be described in detail below.

### BRIEF DESCRIPTION OF THE DRAWINGS

Fig. 1 is a top view of an absorbent article in the form of a diaper comprising an exemplary absorbent core according to the invention with some layers partially removed;
Fig. 2 is a transversal cross-section of the diaper of Fig. 1;
Fig. 3A is a top view of the exemplary absorbent core of the diaper of Fig. 1 taken in isolation wherein the top side of the core wrap is entirely colored;
Fig. 3B is a top view of the exemplary absorbent core of the diaper of Fig. 1 taken in isolation wherein an area of the top side of the core wrap is colored;
Fig. 3C is a top view of the exemplary absorbent core of the diaper of Fig. 1 taken in isolation wherein areas of the top side of the core wrap at the substantially absorbent material free areas are colored;
Fig. 4 is a transversal cross-section of the absorbent core of Fig. 3A, 3B or 3C;
Fig. 5 is a longitudinal cross-section of the absorbent core of Fig. 3A, 3B or 3C;
Fig. 6 is a transversal cross-section of the absorbent article of Fig. 1 taken at the same point as Fig. 2 where channels have formed as a result the diaper being loaded with liquid bodily exudates;
Fig. 7 is a transversal cross-section of the absorbent article of Fig. 1 taken at the same point as Fig. 2 where the core wrap bond(s) completely open such the channels have disappeared as a result the diaper being fully loaded with liquid bodily exudates;
Fig. 8A is an outside perspective view of a fastenable absorbent article as when it is worn by a user and before the absorbent article has been loaded by any liquid bodily exudates;
Fig. 8B is an outside perspective view of the fastenable absorbent article as when it is worn by a user and after the absorbent article has been loaded by liquid bodily exudates.
Fig. 8C is an outside perspective view of the fastenable absorbent article as when it is worn by a user and when the fastenable absorbent article has reached its full capacity.

### DETAILED DESCRIPTION OF THE INVENTION

### Definitions

The term "absorbent article" as used herein refers to disposable products such as diapers, training pants, and the like which are placed against or in proximity to the body of the wearer to absorb and contain the various liquid bodily exudates discharged from the body. Typically these absorbent articles comprise a topsheet, backsheet, an absorbent core and optionally an acquisition layer and/or distribution layer and typically other components, with the absorbent core normally placed between the backsheet and the acquisition system or topsheet.

The term "absorbent core" as used herein refers to a component, which is placed or is intended to be placed within an absorbent article and which comprises an absorbent material enclosed in a core wrap. The term "absorbent core" does not cover an acquisition or distribution layer or any other component of an absorbent article which is not either an integral part of the core wrap or placed within the core wrap. The absorbent core is typically the component of an absorbent article which comprises all, or at least the majority of, superabsorbent polymer and has the most absorbent capacity of all the components of the absorbent article.

The term "diaper" as used herein refers to an absorbent article that is intended to be worn by a wearer about the lower torso to absorb and contain liquid bodily exudates discharged from the body. Diapers may be worn by infants (e.g. babies or toddlers) or adults. They may be provided with fastening elements.

The term "pant" as used herein refers to an absorbent article having fixed edges, a waist opening and leg openings designed for infant or adult wearers. A pant-type absorbent article is placed in position on the wearer by inserting the wearer's legs into the leg openings and sliding the pant-type absorbent article into position about the wearer's lower torso. A pant may be preformed by any suitable technique including, but not limited to, joining together portions of the absorbent article using refastenable and/or non-refastenable bonds (e.g., seam, weld, adhesive, cohesive bond, fastener, etc.). A pant may be preformed anywhere along the circumference of the article (e.g., side fastened, front waist fastened). While the term "pant" or "pants" are used herein, pants are also commonly referred to as "closed diapers", "prefastened diapers", "pull-on diapers", "training pants" and "diaper-pants".

The term "substantially free of absorbent material" or "substantially absorbent material free" as used herein means that the basis weight of the absorbent material is at least less than 10%, in particular less than 5% or 2%, of the average basis weight of the absorbent material in the rest of the absorbent core.

The term "nonwoven web" as used herein means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m² or gsm).

The term "superabsorbent polymers" (herein abbreviated as "SAP") as used herein refer to absorbent materials which are cross-linked polymeric materials that can absorb at least 10 times their weight of an aqueous 0.9% saline solution as measured using the Centrifuge Retention Capacity (CRC) test (EDANA method WSP 241.2-05E). The SAP of the invention may in particular have a CRC value of more than 20 g/g, or more than 25 g/g, or from 20 to 50 g/g, or from 20 to 40 g/g, or 25 to 35 g/g. The SAP useful in the invention includes a variety of water-insoluble, but water-swellable polymers capable of absorbing large quantities of liquid bodily exudates.

The term "color" as used herein refers to the visual perceptual property corresponding in humans to the categories called red, blue, green and others. As used herein, white is not a color. Color derives from the spectrum of light (distribution of light power versus wavelength) interacting in the eye with the spectral sensitivities of the light receptors. Color can include the CMY primary colors (Cyan, Magenta and Yellow).

The term "black" as used herein refers to a color which absorbs wavelengths in the entire spectral region of from about 380 nm to about 740 nm.

The term "blue" or "blue-violet" as used herein refers to a color which has a local maximum reflectance in the spectral region of from about 390 nm to about 490 nm.

The term "green" as used herein refers to a color which has a local maximum reflectance in the spectral region of from about 491 nm to about 570 nm.

The term "red" as used herein refers to a color which has a local maximum reflectance in the spectral region of from about 621 nm to about 740 nm.

The term "cyan" as used herein refers to a primary color which is obtained by the addition of the blue and green light and has a local maximum reflectance in the spectral region of from about 390 nm to about 490 nm and 491 nm to about 570 nm.

The term "magenta" as used herein refers to a primary color which is obtained by the addition of the blue and red light and has a local maximum reflectance in the spectral region of from about 390 nm to about 490 nm and 621 nm to about 740 nm.

The term "Yellow" as used herein refers to a primary color which has a local maximum reflectance in the spectral region of from about 560 nm to about 590 nm.

The term "dye" as used herein refers to a colored substance that has an affinity to a substrate to which it is being applied. The dye is generally a liquid containing coloring matter in an aqueous solution which is applied to the substrate, and may require a mordant to improve the fastness of the dye on the fiber.

The term "pigment" as used herein refers to a material that changes the color of reflected or transmitted light as the result of wavelength-selective absorption. In contrast with a dye, a pigment generally is insoluble, and has no affinity for a substrate. The pigment remains in the form of fine particles dispersed in the substrate.

The term "ink" as used herein refers to a fluid or viscous substance that contains insoluble pigments suspended in a liquid or dyes. The ink typically forms a coating on the surface of the substrate rather than penetrate the entire substrate.

The term "color printing" as used herein refers to the method of providing color prints using any types of color or the reproduction of an image or text in color.

"Comprise," "comprising," and "comprises" are open ended terms, each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essential of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "advantageously" and the likes also qualify features which are not intended to limit the scope of the claims unless specifically indicated to do so.

Unless indicated otherwise, the description refers to the absorbent core and absorbent article before use (i.e. dry, and not loaded with a liquid bodily exudate) and conditioned at least 24 hours at 21 °C +/- 2 °C and 50% +/- 20% Relative Humidity (RH).

### General description of the absorbent article 20

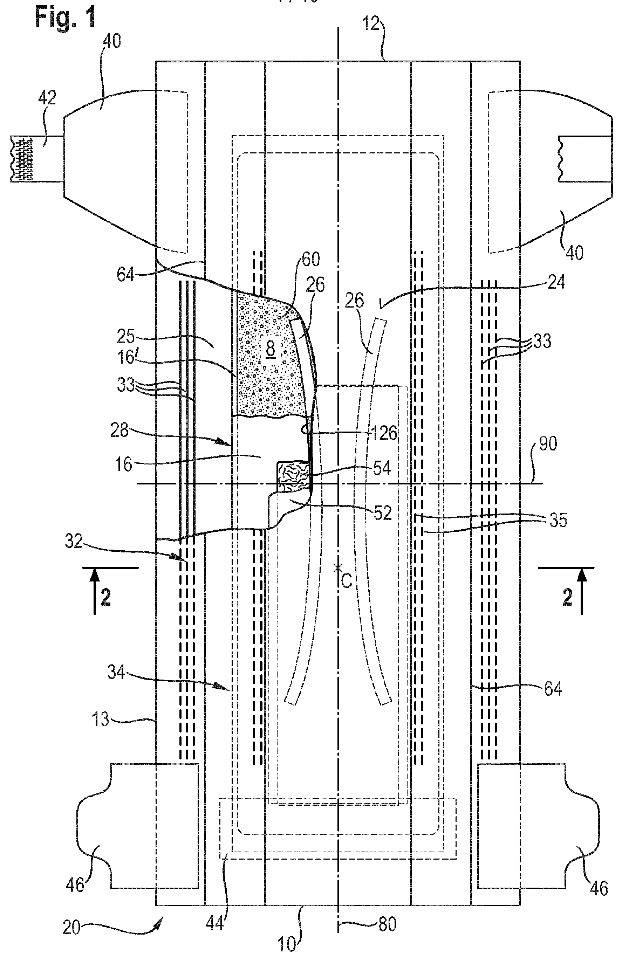

An exemplary absorbent article 20 in which the absorbent core 28 of the invention can be used is an infant taped diaper 20 as represented in Fig. 1. Fig. 1 is a top plan view of the exemplary diaper 20, in a flat-out state, with portions of the structure being cut-away to more clearly show the construction of the diaper 20. This diaper 20 is shown for illustration purpose only as the invention may be used for making a wide variety of diapers or other absorbent articles.

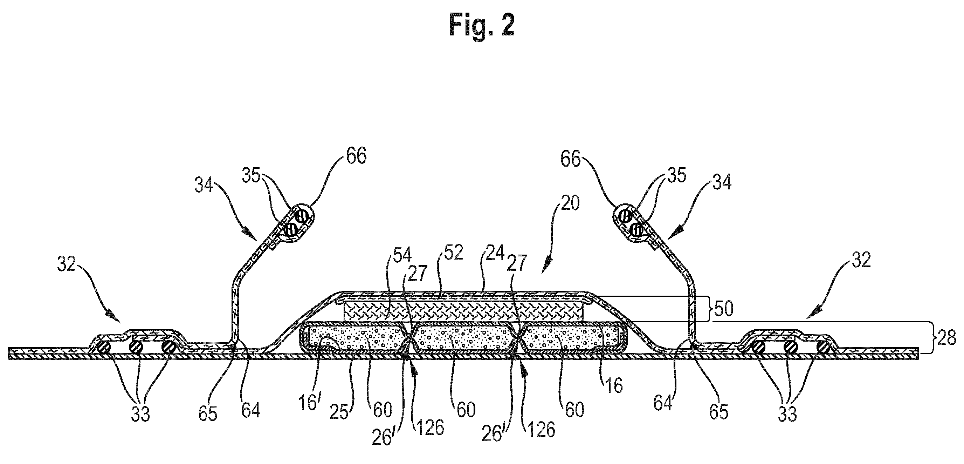

The absorbent article 20 comprises a liquid permeable topsheet 24, a liquid impermeable backsheet 25, an absorbent core 28 between the topsheet 24 and the backsheet 25. The absorbent article 20 comprises a front edge 10, a back edge 12, and two longitudinal side edges 13. The front edge 10 is the edge of the absorbent article 20 which is intended to be placed towards the front of the user when worn, and the back edge 12 is the opposite edge. The absorbent article 20 may be notionally divided by a longitudinal axis 80 extending from the front edge to the back edge of the absorbent article 20 and dividing the absorbent article 20 in two substantially symmetrical halves relative to this axis, when viewing the absorbent article 20 from the wearer facing side in a flat out configuration, as exemplarily shown in Fig. 1. The absorbent article 20 may also comprise further typical components such as an acquisition layer 52 and/or a distribution layer 54 (collectively referred to as acquisition-distribution system "ADS", designated as 50 in Fig. 2), and elasticized gasketing cuffs 32 present between the topsheet 24 and the backsheet 25 and upstanding barrier leg cuffs 34. Figs. 1-2 also show other typical taped diaper components such as a fastening system comprising fastening tabs 42 attached towards the back edge 12 of the absorbent article 20 and cooperating with a landing zone 44 towards the front edge 10 of the absorbent article 20. The absorbent article 20 may also comprise other typical components, which are not represented in the Figures, such as a back elastic waist feature, a front elastic waist feature, transverse barrier cuff(s), a lotion application, etc.

If some part of the absorbent article 20 is under tension due to elasticized components, the absorbent article 20 may be typically flattened using clamps along the periphery of the absorbent article 20 and/or a sticky surface, so that the topsheet 24 and backsheet 25 can be pulled taut so as to be substantially flat. The absorbent article 20 can also be notionally divided by a transversal axis 90 in a front region and a back region of equal length measured on the longitudinal axis, when the absorbent article 20 is in such a flat state. The absorbent article's transversal axis 90 is perpendicular to the longitudinal axis 80 and placed at half the length of the absorbent article 20. The length of the absorbent article 20 can be measured along the longitudinal axis 80 from the front edge 10 to the back edge 12 of the absorbent article 20.

The topsheet 24, the backsheet 25, the absorbent core 28 and the other absorbent article components may be assembled in a variety of well known configurations, in particular by adhesive bonding and/or heat and/or pressure embossing. Exemplary diaper assemblies are for example generally described in US3,860,003, US5,221,274, US5,554,145, US5,569,234, US5,580,411, and US6,004,306. The absorbent article 20 is preferably thin. The absorbent article 20 may be advantageously thin at the intersection of the longitudinal and transversal axis, for example with a caliper of from 1.0 mm to 8.0 mm, in particular from 1.5 mm to 6.0 mm, as measured using the Absorbent Article Caliper Test described below.

Some components of the absorbent article 20 will now be discussed in more details.

### Absorbent core 28

The absorbent core 28 of the invention comprises an absorbent material 60 with a high amount of superabsorbent polymers enclosed within a core wrap. The core wrap comprises a top side 16 facing the topsheet 24 and a bottom side 16' facing the backsheet 25. The absorbent material 60 may comprise at least 80% of SAP by weight of the absorbent material 60 up to substantially 100% of SAP, by total weight of the absorbent material 60. In that respect, the absorbent core 28 may comprise from 5 g to 60 g of SAP, in particular from 10 g to 50 g of SAP. The core wrap is not considered as an absorbent material 60 for the purpose of assessing the percentage of SAP in the absorbent core 28. The absorbent material 60 defines an absorbent material deposition area 8 as seen when the absorbent core 28 is placed substantially flat. As used herein, the term "absorbent core" does not include the topsheet 24, the backsheet 25 and (if present) an acquisition-distribution system or layer 50 which is no integral part of the absorbent core 28, in particular which is not placed within the core wrap.

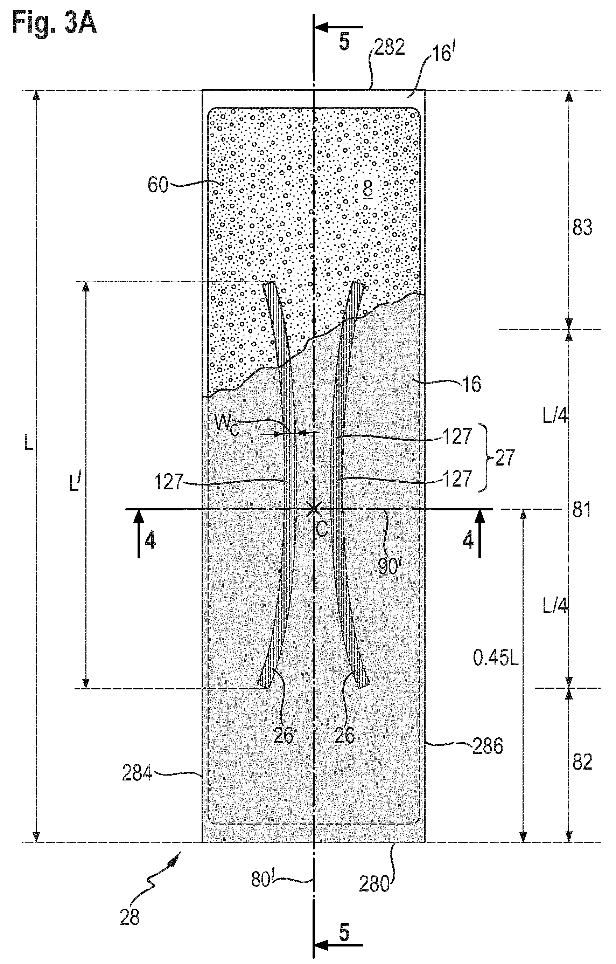

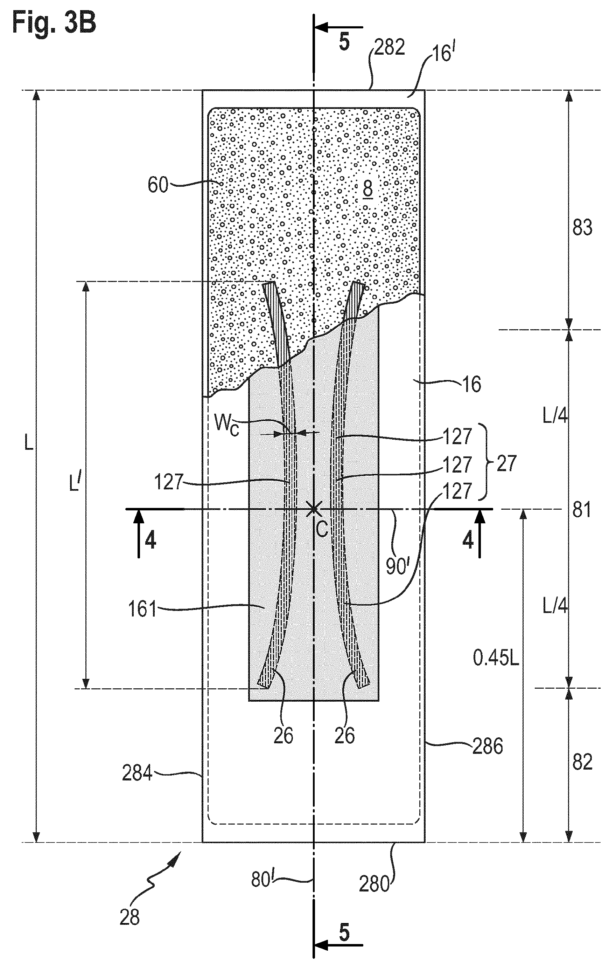

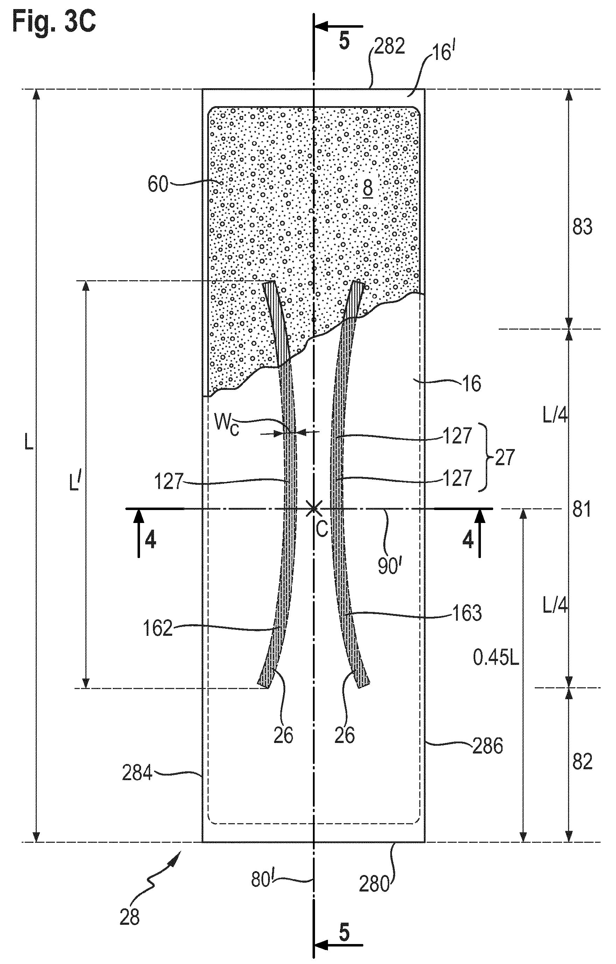

The exemplary absorbent core 28 of the absorbent article of Fig. 1 is shown in isolation in Figs. 3-5. The absorbent core 28 of the invention comprises a front edge 280, a back edge 282 and two longitudinal edges 284, 286 joining the front edge 280 and the back edge 282, a longitudinal axis 80' and a transversal axis 90'. The front edge 280 of the absorbent core 28 is the edge of the absorbent core 28 intended to be placed towards the front edge 10 of the absorbent article 20. Typically the absorbent material 60 will be advantageously distributed in higher amount towards the front edge 280 than towards the back edge 282 as more absorbency is required at the front. Typically the front and back edges 280, 282 of the absorbent core 28 are shorter than the longitudinal edges 284, 286 of the absorbent core 28. The absorbent core 28 may also comprise a top side 288 and a bottom side 290. The top side 288 of the absorbent core 28 is the side intended to be placed towards the topsheet 24 and the bottom side 290 the side intended to be placed towards the backsheet 25 in the finished absorbent article 20. The top side 288 of the absorbent core 28 is typically more hydrophilic than the bottom side 290 of the absorbent core 28.

The absorbent core 28 may consist essentially of, or consist of, the core wrap, the absorbent material 60 and optionally adhesive. In that case, the top side 288 of the absorbent core 28 coincides with the top side 16 of the core wrap. Also, the bottom side 290 of the absorbent core 28 coincides with the bottom side 16' of the core wrap.

The crotch point C may be defined by the intersection of the longitudinal axis 80' of the absorbent core 28 and the transversal axis 90' of the absorbent core 28. The width of the absorbent core 28 at the crotch point C may in particular be of from 45 mm to 200 mm, or from 50 mm to 150 mm.

The absorbent core 28 may be notionally divided by the longitudinal axis 80' extending from the front edge 280 to the back edge 282 of the absorbent core 28 and dividing the absorbent core 28 in two substantially symmetrical halves relative to this axis, when viewing the absorbent core 28 from the topside in a flat out configuration, as exemplarily shown in Fig. 3A-C. Typically the longitudinal axis 80' of the absorbent core 28 and the longitudinal axis 80 of the absorbent article 20 in which the absorbent core 28 is intended to be placed will be contiguous, when viewed from the top as in Fig. 1. The transversal axis 90' of the absorbent core 28, is perpendicular to the longitudinal axis and is passing through the crotch point C of the absorbent core 28. The crotch point C is the point of the absorbent core 28 placed at a distance of 0.45 of L from the front edge of the absorbent core 28, L being the length of the absorbent core 28 as measured from its front edge 280 to its back edge 282 on the longitudinal axis 80', as illustrated in Fig. 3A-C. The full length L of the absorbent core 28 is measured from the front edge 280 to the back edge 282 of the absorbent core 28 along its longitudinal axis 80' and also includes the region of the core wrap which does not enclose the absorbent material 60, in particular at the front and back end seals when present. The length L of the absorbent core 28 may be of at least 320 mm, for example from 320 mm to 600 mm, for typical diaper applications.

The crotch region 81 is defined herein as the region of the absorbent core 28 extending from the transversal axis 90' of the absorbent core 28, i.e. at the level of the crotch point C, towards the back edge 282 and front edge 280 of the absorbent core 28 by a distance of a quarter of L (L/4) in both directions for a total length of L/2. The front region 82 and back region 83 of the absorbent core 28 are the remaining regions of the absorbent material deposition area 8 towards the front and back edges 280, 282 of the absorbent core 28 respectively.

The absorbent core 28 may also advantageously achieve an SAP loss of no more than about 70%, 60%, 50%, 40%, 30%, 20%, 10% according to the Wet Immobilization Test described in WO2010/0051166A1. Further aspects of the absorbent core 28 will now be described in further details.

By "absorbent material" it is meant a material which has at least some absorbency and/or liquid retaining properties, such as SAP, cellulosic fibers as well as some hydrophilically treated synthetic fibers. Typically, adhesives used in making absorbent cores have no absorbency properties and are not considered as absorbent material. The SAP content may be higher than 80%, for example at least 85%, at least 90%, at least 95% and even up to and including 100% of the weight of the absorbent material 60 contained within the core wrap. This high SAP content may provide a relatively thin absorbent core 28 compared to conventional absorbent core typically comprising between 40-60% SAP and the rest of cellulosic fibers. The absorbent material 60 of the invention may in particular comprises less than 10% weight percent, or less than 5% weight percent, or even be substantially free of natural and/or synthetic fibers. The absorbent material 60 may advantageously comprise little or no (airfelt) cellulosic fibers, in particular the absorbent core 28 may comprise less than 15%, 10%, or 5% (airfelt) cellulosic fibers by weight of the absorbent core 28, or even be substantially free of cellulose fibers. The absorbent core 28 of the invention may be relatively thin and thinner than conventional airfelt cores.

The absorbent core 28 of the invention may further comprise adhesive for example to help immobilizing the SAP within the core wrap and/or to ensure integrity of the core wrap, in particular when the core wrap is made of one or more substrates. The core wrap will typically extend to a larger area than strictly needed for containing the absorbent material 60 within.

Absorbent cores 28 comprising relatively high amount of SAP with various absorbent core designs have been proposed in the past, see for example in US5,599,335 (Goldman), EP1,447,066 (Busam), WO95/11652 (Tanzer), US2008/0312622A1 (Hundorf), WO2012/052172 (Van Malderen) and WO2012/170778 (Rosati et al., see also WO2012/170779, WO2012/170781 and WO2012/1708008). The absorbent core may be thin, for example having a thickness not exceeding 5 mm, e.g. from 1 to 4 mm, as measured with the Dry Absorbent Core Caliper Test disclosed therein.

### Absorbent material deposition area 8

The absorbent core 28 may comprise an absorbent material deposition area 8 defined by the periphery of the layer formed by the absorbent material 60 within the core wrap, as seen from the top when the absorbent core 28 is laid flat, as illustrated in Fig. 3A-C. The absorbent material 60 may be applied continuously or discontinuously in the absorbent material deposition area 8. If substantially absorbent material free areas 26 or junction areas between pockets or stripes of absorbent material 60 are present, these are considered to be part of the absorbent material deposition area 8, for example for the purpose of measuring the width or the length L of the absorbent material deposition area 8.

The shape of the absorbent material deposition area 8 can vary, in particular it can be rectangular as shown in Fig. 3A-C or shaped with a so-called "dog bone" or "hourglass" shape, which shows a tapering along its width at least in the crotch region 81 of the absorbent material deposition area 8. When shaped (non-rectangular), the absorbent material deposition area 8 may have a relatively narrow width in the crotch region 81 of the absorbent core 28 as this may provide for example better wearing comfort in the finished absorbent article 20 incorporating the absorbent core 28. The absorbent material deposition area 8 may thus have a width (as measured in the transversal direction perpendicular to the longitudinal axis 80') at its narrowest point which is less than about 100 mm, 90 mm, 80 mm, 70 mm, 60 mm or even less than about 50 mm. This narrowest width may typically be in the crotch region 81 and may further be for example at least 5 mm, or at least 10 mm, or at least 20 mm smaller than the maximum width of the absorbent material deposition area 8 at its largest point in the front region 82 and/or back region 83 of the absorbent core 28.

The basis weight (amount deposited per unit of area) of the absorbent material 60 may also be varied along the absorbent material deposition area 8 to create a profiled distribution of the absorbent material 60 in the longitudinal direction, in the transversal direction, or both directions of the absorbent core 28. Hence the basis weight of the absorbent material 60 may vary along the longitudinal axis 80' of the absorbent core 28, as well as along the transversal axis 90', or any axis parallel to any of these axes. The basis weight of absorbent material 60 in area of relatively high basis weight such as the crotch point C may thus be for example at least 10%, or 20%, or 30%, or 40%, or 50% higher than in an area of relatively low basis weight. In particular the absorbent material 60 present in the absorbent material deposition area 8 at the level of the crotch point C may have more SAP per unit of area deposited as compared to any other area of the front region 82 or back region 83 of the absorbent material deposition area 8. The basis weight of the SAP may be at least 10%, or 20%, or 30%, or 40%, or 50% higher at the crotch point C of the absorbent core 28 than at another point of the absorbent material deposition area 8 on the longitudinal axis 80', in particular in the front or back region 82, 83 of the absorbent core 28.

### Core wrap

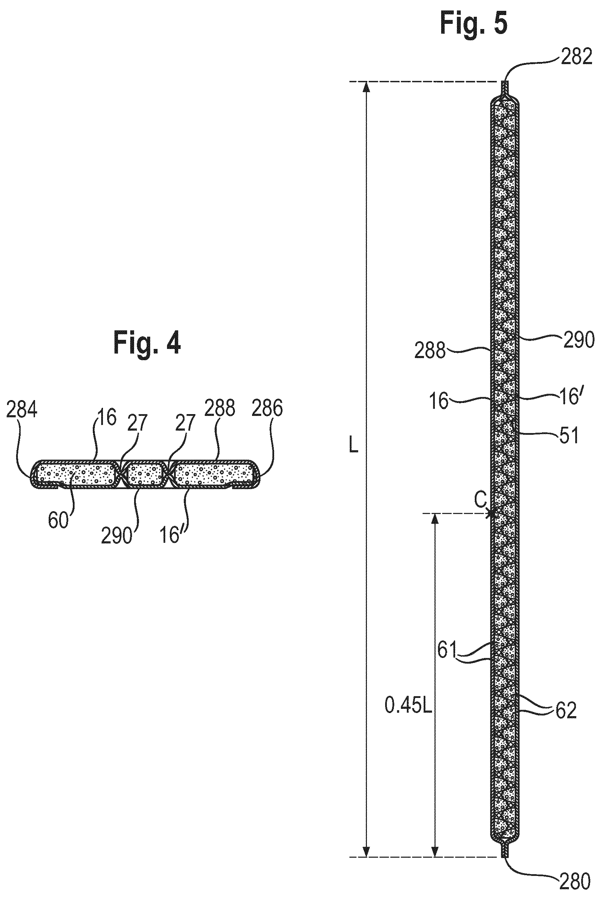

The core wrap comprises the top side 16 facing the topsheet 24 and the bottom side 16' facing the backsheet 25. The core wrap may be made of a single substrate folded around the absorbent material 60. The core wrap may be made of two (or more) substrates which are attached to another. Typical configurations are the so-called C-wrap and/or sandwich wrap. In a C-wrap, as exemplarily shown in Figs. 2 and 4, the longitudinal and/or transversal edges of one of the substrate are folded over the other substrate to form flaps. These flaps are then bonded to the external surface of the other substrate, typically by bonding with an adhesive.

The core wrap may be formed by any materials suitable for receiving and containing the absorbent material 60. Typical substrate materials used in the production of conventional absorbent cores may be used, in particular paper, tissues, films, wovens or nonwovens, or laminate of any of these. The core wrap may in particular be formed by a nonwoven web, such as a carded nonwoven, spunbond nonwoven ("S") or meltblown nonwoven ("M"), and laminates of any of these. For example spunmelt polypropylene nonwovens are suitable, in particular those having a laminate web SMS, or SMMS, or SSMMS, structure, and having a basis weight range of about 5 gsm to 15 gsm. Suitable materials are for example disclosed in US7,744,576, US2011/0268932A1, US2011/0319848A1 or US2011/0250413A1. Nonwoven materials provided from synthetic fibers may be used, such as PE, PET and in particular PP.

When the core wrap are made of two (or more) substrates, the two (or more) substrates of the core wrap may be made of the same type of material, or may be made of different materials or one of the substrate may be treated differently than the other to provide it with different properties. As the polymers used for nonwoven production are inherently hydrophobic, they are preferably coated with hydrophilic coatings if placed on the fluid receiving side of the absorbent core 28. It is advantageous that the top side 16 of the core wrap is more hydrophilic than the bottom side 16' of the core wrap. A possible way to produce nonwovens with durably hydrophilic coatings is via applying a hydrophilic monomer and a radical polymerization initiator onto the nonwoven, and conducting a polymerization activated via UV light resulting in monomer chemically bound to the surface of the nonwoven. An alternative possible way to produce nonwovens with durably hydrophilic coatings is to coat the nonwoven with hydrophilic nanoparticles, e.g. as described in WO02/064877.

The top side 16 of the core wrap may be sealed to the bottom side 16' of the core wrap at least partially along all the edges of the absorbent core 28. The term "seal" is to be understood in a broad sense. The seal does not need to be continuous along the whole periphery of the core wrap but may be discontinuous along part or the whole of it, such as formed by a series of closely spaced apart seal points on a line. Typically a seal may be formed by adhesive bonding and/or thermal bonding.

When the core wrap is formed by two substrates, one seal per edge of the absorbent core 28 may typically be used to enclose the absorbent material 60 within the core wrap. This is exemplified in the Figs. 4 and 5. As shown in Figure 4, for example, a first substrate including the top side 16 of the core wrap may be placed on one side of the absorbent core 28 (the top side 288 of the absorbent core 28 as represented therein) and extends around the absorbent core's longitudinal edges to at least partially wrap the opposed bottom side 290 of the absorbent core 28. A second substrate including the bottom side 16' of the core wrap can be present between the wrapped flaps of the first substrate of the core wrap and the absorbent material 60 of the absorbent core 28. The flaps of the first substrate of the core wrap may be adhesively bonded to the second substrate of the core wrap to provide a strong seal. This so called C-wrap construction can provide benefits such as improved resistance to bursting in a wet loaded state compared to a sandwich seal. A front edge and back edge of the core wrap may then also be sealed for example by bonding the first substrate and second substrate of the core wrap, flat to another to provide more complete enclosure of the absorbent material 60 across the whole of the periphery of the absorbent core 28. It can be advantageous to use the C-wrap at least on the longitudinal edges 284, 286 of the absorbent core 28 which are longer than the front and end edges 280, 282 of the absorbent core 28. In the so-called sandwich construction, the first and second substrates of the core wrap may also extend outwardly on all edges of the absorbent core 28 and be sealed flat along the whole or parts of the periphery of the absorbent core 28 typically by adhesive bonding and/or heat/pressure bonding. Typically neither first nor second substrates of the core wrap need to be shaped, so that they can be rectangularly cut for ease of production but of course other shapes are possible.

Alternatively, the core wrap may also be formed by a single substrate which may enclose as in a parcel wrap the absorbent material 60 and be for example sealed along the front edge and back edge 280, 282 of the absorbent core 28 and one longitudinal seal.

### Substantially absorbent material free areas 26 and colored areas 126

The absorbent core 28 comprises one or more substantially absorbent material free area(s) 26 which is/are substantially free of absorbent material 60 and through which a portion of the top side 16 of the core wrap is attached by one or more core wrap bond(s) 27 to a portion of the bottom side 16' of the core wrap.. In particular, there can be no absorbent material 60 in these areas. Minimal amount such as contaminations with absorbent material 60 that may occur during the making process are not considered as absorbent material 60. The one or more substantially absorbent material free area(s) 26 is/are advantageously confined by the absorbent material 60, which means that the substantially absorbent material free area(s) 26 do(es) not extend to any of the edge of the absorbent material deposition area 8.

If the substantially absorbent material free area 26 extends to any of the edges of the absorbent material deposition area 8, each substantially absorbent material free area 26 may have areas of absorbent material 60 on either side of each substantially absorbent material free area 26.

The portions of the top side 16 and the bottom side 16' of the core wrap may be attached together continuously along the substantially absorbent material free area(s) 26. However, one or more core wrap bonds 27 along the substantially absorbent material free area(s) 26 may also be discontinuous (intermittent) such as series of point bonds. The core wrap bond(s) (27) may be provided by known attachment means, such as adhesive bonding, pressure bonding, ultrasonic bonding or heat bonding, dynamic mechanical bonding or combination thereof.

The attachment of the portions of the top side 16 and the bottom side 16' of the core wrap may be provided by one or more adhesive material(s), in particular one or more layers of adhesive and/or one or more layers of fibrous adhesive material, if present in the absorbent core 28. These adhesives may therefore serve the dual function of immobilizing the absorbent material 60 and attach the top side 16 to the bottom side 16' of the core wrap together at one or more substantially absorbent material free area(s) 26.

For example, the attachment of the portions of the top side 16 to the bottom side 16' of the core wrap may include a uniform continuous layer of adhesive, a patterned layer of adhesive, or an array of separate lines, spirals, or spots of adhesive. Suitable attachment means comprise an open pattern network of filaments of adhesive as disclosed in US4,573,986. Other suitable attachment means include several lines of adhesive filaments which are swirled into a spiral pattern, as is illustrated by the apparatus and methods shown in US3,911,173, US4,785,996; and US4,842,666. Adhesives which have been found to be satisfactory are manufactured by H. B. Fuller Company of St. Paul, Minnesota and marketed as HL-1620 and HL 1358-XZP.

The basis weight of the adhesive applied between the top side 16 and the bottom side 16' of the core wrap may be homogeneous across the core wrap bond(s) 27. The basis weight of the adhesive applied between the top side 16 and the bottom side 16' of the core wrap across the core wrap bond(s) 27 may be from 1 to 20 gsm or from 5 to 15 gsm.

Advantageously, the portion of the top side 16 and the portion of the bottom side 16' of the core wrap may be attached together at one or more substantially absorbent material free area(s) 26 by an adhesive comprising a plurality of adhesive lines 127 which are substantially parallel to the longitudinal axis 80' of the absorbent core 28 (shown in Fig. 3A-C). The plurality of adhesive lines 127 may be provided by an adhesive slot apparatus. The plurality of adhesive lines 127 may represent the one or more core wrap bond(s) 27.

The following examples of the shape and size of the substantially absorbent material free area(s) 26 are not limiting. In general, the core wrap bond(s) 27 may have the same outline but be slightly smaller than the substantially absorbent material free area(s) 26 due to the tolerance required in some manufacturing process. The substantially absorbent material free area(s) 26 may be present within the crotch region 81 of the absorbent article 20. In particular, the substantially absorbent material free area(s) 26 may be extending longitudinally parallel to the longitudinal axis 80' of the absorbent core 28 and/or transversely parallel to the transversal axis 90' of the absorbent core 28. Fig. 3A-C show two longitudinally extending substantially absorbent material free areas 26 as an example. The absorbent core 28 may also comprise more than two substantially absorbent material free areas 26, for example at least 3, or at least 4 or at least 5 or at least 6.

The absorbent core 28 may comprise at least 2 substantially absorbent material free areas 26 symmetrically disposed on both sides of the longitudinal axis 80' of the absorbent core 28. Shorter substantially absorbent material free area(s) 26 may also be present, for example in the back region or the front region 83, 82 of the absorbent core 28, as seen for example in the Figures of WO2012/170778.

The substantially absorbent material free area(s) 26 may be at least partially oriented in the longitudinal direction of the absorbent article 20. This means typically that each substantially absorbent material free area 26 extends more in the longitudinal direction than in the transverse direction, and typically at least twice as much in the longitudinal direction than in the transverse direction. The substantially absorbent material free area(s) 26 may have a length L' projected on the longitudinal axis 80' of the absorbent core 28 that is at least 10% of the length L of the absorbent core 28.

The substantially absorbent material free area(s) 26 may be straight and completely oriented longitudinally and parallel to the longitudinal axis but also may be curved or have one or more curved portions. In particular some or all these substantially absorbent material free area(s) 26, in particular these substantially absorbent material free area(s) 26 present in the crotch region, may be concave towards the longitudinal axis 80', as for example represented in Fig. 3A-C for the pair of substantially absorbent material free areas 26. The radius of curvature may typically be at least equal (and preferably at least 1.5 or at least 2.0 times this average transverse dimension) to the average transverse dimension of the absorbent material deposition area 8; and also straight but with an angle of (e.g. from 5°) up to 30°, or for example up to 20°, or up to 10° with a line parallel to the longitudinal axis 80'. The radius of curvature may be constant for each substantially absorbent material free area 26, or may vary along its length. This may also includes substantially absorbent material free area(s) 26 with an angle therein, provided said angle between two parts of a channel is at least 120°, preferably at least 150°. These substantially absorbent material free area(s) 26 may also be branched, for example a central substantially absorbent material free area superposed with the longitudinal axis 80' in the crotch region which branches towards the back and/or towards the front of the absorbent article 20.

There may be no substantially absorbent material free area(s) 26 that coincides with the longitudinal axis 80' of the absorbent core 28. When present as one of symmetrical pair(s) relative to the longitudinal axis 80' of the absorbent core 28, the substantially absorbent material free area(s) 26 may be spaced apart from one another over their whole longitudinal dimensions. Generally, the smallest spacing distance may be for example at least 5 mm, or at least 10 mm, or at least 16 mm.

Furthermore, in order to reduce the risk of liquid bodily exudate leakages, the substantially absorbent material free area(s) 26 advantageously do not extend up to any of the edges of the absorbent material deposition area 8, and are therefore surrounded by and fully encompassed within the absorbent material deposition area 8 of the absorbent core 28. Typically, the smallest distance between a substantially absorbent material free area 26 and the closest edge of the absorbent material deposition area 8 is at least 5 mm.

The substantially absorbent material free area(s) 26 may have a width Wc along at least part of its length which is at least 2 mm, or at least 3 mm or at least 4 mm, up to for example 20 mm, or 16 mm or 12 mm. The width Wc of the substantially absorbent material free area(s) 26 may be constant through substantially its whole length or may vary along its length.

The top side 16 of the core wrap is at least colored in the portion of the top side 16 of the core wrap which is attached by one or more core wrap bond(s) 27 to the portion of the bottom side 16' of the core wrap.

The top side 16 of the core wrap may comprise one or more colors. The one or more colors may have different shades of colors. Colors may be selected from the group consisting of black, blue, cyan, green, magenta, red, yellow and any combinations thereof. When a substrate of the core wrap comprises one or more colors having different shades of colors, it is known that a shade of color shall be defined in terms of chroma, hue and lightness according to the Commission Internationale de l'Eclairage L*a*b* Color Space as defined and measured according to the spectrophotometric Color Test Method in WO2012/121954.

By modifying the chroma, the hue and/or the lightness, one or more variation of colors may be created and may be imparted to the top side 16 of the core wrap, as long as the color is visible to the human eyein order to create a visual dryness indicia. In order to be perceived by the consumer, darker colors may be thus preferred within the available gamut of colors.

Alternatively, the top side 16 of the core wrap may comprise one or more portions. Each portion of the top side 16 of the core wrap, may have one color or one single variation of color. The color and/or the single variation of colors of each portion may be different between them.

One or more colors and/or one or more variations of color may be imparted to the top side 16, in at least the portion of the top side 16 of the core wrap which is attached by one or more core wrap bond(s) 27 to the portion of the bottom side 16' of the core wrap by any color technique such as pigmentation, printing, coating and impregnating.

One or more colors and/or one or more variations of color may be imparted to the 16 of the core wrap or the substrate forming the top side 16 of the core wrap by color pigmentation.

As is known in the art, pigments are colored, black, white or fluorescent particulate organic and inorganic solids which are usually insoluble in, and essentially physically and chemically unaffected by the vehicle or substrate in which they are incorporated. They alter appearance by selective absorption and/or by scattering of light. The term "colored pigment" encompasses any pigments suitable for imparting a non-white color to a substrate, e.g. a nonwoven material. This term therefore does not include "white" pigments such as TiO₂ which are typically added to the layers of conventional absorbent articles to impart them with a white appearance. Example of colored pigment is carbon black however it may be preferred to use non-black pigments which provide a more subtle tint in the pastel range.

Pigments are usually dispersed in vehicles or substrates for application, as for instance in inks, paints, plastics or other polymeric materials. Pigments retain a crystal or particulate structure throughout the coloration process. As result of the physical and chemical characteristics of pigments, pigments and dyes differ in their application; when a dye is applied, it penetrates the substrate in a soluble form after which it may or may not become insoluble. When pigments are used to color or opacify a substrate, the finely divided insoluble solids remain throughout the coloration process.

Compounding is the typical process by which color or additives are added to a basic thermoplastics material. This usually involves melting the thermoplastics material then mixing it with the required pigments and/or additive material in an extruder. The polymer melt is then extruded and chopped into pellets as it cools, which can then be used directly by the plastics processor. An associated process is masterbatch. This is where a high concentration of pigment and/or additives are dispersed in a carrier medium which can then be used directly by the processor in small quantities to pigment or modify the virgin polymer material. The pigment is added at high concentration into the carrier (e.g. polypropylene resin) supplied in pellets. The colored masterbatch is mixed to the matrix made of virgin resin in the nonwoven line at an add-on level needed to reach the desired color on the final material. The pigments may for example be introduced in a polypropylene masterbatch to obtain a pigmented bicomponent nonwoven. An example of suitable colored masterbatch material that can be introduced is Pantone color 270 Sanylen violet PP 42000634 ex Clariant, which is a PP resin with a high concentration of violet pigment. Typically, the amount of pigments introduced by weight of non-woven material after the compounding may be of from 0.3% - 2.5%.

A variety of coating techniques may be used to impart color onto the top side 16 of the core wrap or the substrate forming the top side 16 of the core wrap. Suitable coating techniques are well-known in the art and include, but are not limited to, bead extruders, slot die coaters, spray nozzles, dip tanks, brushes, and combinations thereof. Suitable slot die coaters include the EP11 Applicator available from Nordson Corp., Dawsonville, GA or the MR1300 Slot Die Coater available from ITW Dynatec Americas, Hendersonville, TN. Suitable coatings include, but are not limited to, adhesives, varnishes, latexes, lotions, waxes, and paraffins. The coatings generally will contain a dye, pigment, or combination.

Color may be imparted to a substrate by way of impregnation of a colorant into the substrate. Colorants such as dyes, pigments, or combinations may be impregnated in the formation of substrates such as polymerics, resins, or nonwovens. For example, the colorant may be added to molten batch of polymer during film, fiber, or filament formation.

One or more colors and/or one or more variations of color may be preferably imparted to a substrate by color printing. The substrate forming the top side 16 of the core wrap may be color printed during the manufacture of components of absorbent articles. Various color printing methods may be used to impart color onto a substrate, including, but not limited to, letterpress, flexographic printing, gravure printing, lithographic printing, screen printing and inkjet printing.

Letterpress involves ink or other equivalent material being applied to the top of a raised surface. This surface is pressed against the substrate, thus transferring the ink to the substrate.

Flexographic printing uses a printing plate, often cylindrical, made of rubber, plastic, or other flexible material. Ink is applied to a raised image on the plate. The plate is then placed in contact with the substrate, and ink is transferred to the substrate. Water-based and solvent-based inks are used in flexography.

Gravure printing uses a print cylinder having depressions of varying depths that are etched into the cylinder. This method of printing is performed by partially immersing the etched cylinder (generally about a fourth of the cylinder diameter) into an enclosed fountain or trough of ink. The etched cells, which produce the image, are filled with ink, and the surface the cylinder also becomes coated with ink. As the printing cylinder comes in contact with the substrate, the ink contained within the cells is transferred to the substrate. Gravure is ideal for continuous printing operations and the printing of very long runs.

Lithographic printing, or offset lithography, is a printing method that utilizes surface characteristics on an image carrying offset plate. Offset plates are typically made from a thin paper, plastic, or a metal sheet which once exposed and processed can be wrapped around a cylinder of a press for printing.

Screen printing utilizes a porous screen made from silk or other polymeric material. The screen is attached to a frame. A stencil is produced on the screen either photo-mechanically or manually. The non-printing areas are protected by the stencil. Printing is done on a substrate under the screen by applying a viscous ink to the screen. The ink is forced through the fine openings of the screen with a rubber squeegee or roller.

One or more colors and/or one or more variations of color may be preferably imparted to the substrate forming the top side 16 of the core wrap and thus to the top side 16 of the core wrap by inkjet printing.

Inkjet printing is a non-impact dot-matrix technology where ink droplets are jetted from a small aperture directly to specified positions on a medium to create an image. Inkjet printing may be done on a continuous method or a drop-on-demand method. Inkjet printing techniques are well known in the art as described in Hue. P. Le, Progress and Trends in Ink-Jet Printing Technology, Journal of Imagining Science and Technology, Vol. 42, pages 49-62.

Color printing may also be carried out with a central impression printing, in-line printing, multi-stage printing system or contact printing system. Central impression printing may be used to provide ink to the substrate forming the top side 16 of the core wrap. Exemplary central impression printing methods and apparatus are described in US6,220,156; US6,283,024; and US5,083,511. In-line printing may be used to provide ink to the substrate forming the top side 16 of the core wrap. Exemplary in-line printing methods and apparatus are described in US6,587,133; US6,026,748; and US5,331,890. Color printing may be preferably carried out with a multi-stage printing system, such as those exemplified in US5,638,752, US6,026,748, and US5,331,890.

Advantageously for cost reasons and process easiness, the top side 16 of the core wrap may be made of a single substrate which may be a pigmented nonwoven. The top side 16 of the core wrap may be thus entirely colored. Fig. 3A shows an absorbent core 28 wherein a top side 16 of the core wrap is entirely colored.

Alternatively, the top side 16 of the core wrap may include a colored area 161 which encompasses the one or more substantially absorbent material free area(s) 26. The colored area 161 of the top side 16 of the core wrap may be larger than the one or more substantially absorbent material free area(s) 26 but smaller than the whole top side 16 of the core wrap. Each colored area may be provided by color printing, preferably by inkjet printing. Fig. 3B shows how the colored area 161 of the top side 16 of the core wrap encompasses two substantially absorbent material free area(s) 26.

Alternatively, the top side 16 of the core wrap may include a colored area corresponding to each substantially absorbent material free area(s) 26. Each colored area may be provided by color printing, preferably by inkjet printing. Figure 3C shows that the top side 16 of the core wrap include two colored areas (162, 163) corresponding to the two substantially absorbent material free area(s) 26.

A portion of the top side 16 of the core wrap is attached to a portion of the bottom side 16' of the core wrap to create one or more core wrap bonds 27 at the one or more substantially absorbent material free area(s) 26. The one or more substantially absorbent material free area(s) 26 includes portions of the top side 16 of the core wrap which are colored. These colored portions of the top side 16 of the core wrap form one or more colored area(s) 126 visible through the exterior of the absorbent article 20.

The backsheet 25 may be positioned tightly to the bottom side 16' of the core wrap. The visibility of the colored area(s) 126 through the backsheet 25 may be advantageously ensured by bonding the backsheet 25 to the bottom side 16' of the core wrap. The backsheet 25 may be bonded to the bottom side 16' of the core wrap at one or more substantially absorbent material free area(s) 26.

In order to ensure that the colored area(s) 126 is/are visible when the consumer is looking at them from the exterior of the absorbent article 20, the bottom side 16' of the core wrap and the backsheet 25 at one or more substantially absorbent material free area(s) 26 may be advantageously transparent. The backsheet 25 may also not comprise any graphics at one or more substantially absorbent material free area(s) 26.

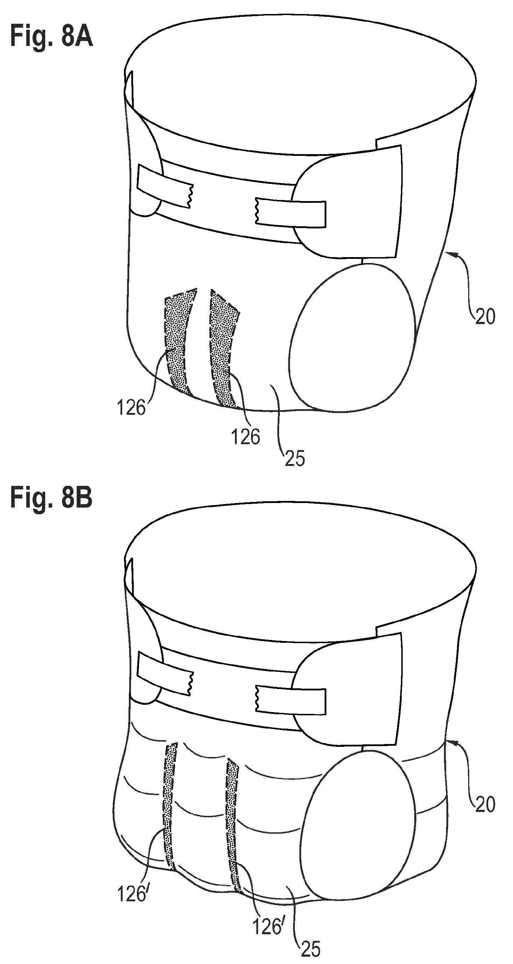

Fig. 8A is an outside perspective view of a front and a side of a fastenable absorbent article 20 when it is worn by a user and before the absorbent article 20 has been loaded by any liquid bodily exudates. Two colored areas 126 are visible from the backsheet 25 to the caregiver.

When the absorbent material 60 swells, one or more core wrap bond(s) 27 at least partially open so that the colored area(s) 126 becomes no longer or less visible from the exterior of the absorbent article 20, i.e. through the bottom side 16' of the core wrap and the backsheet 25, as it will described in detail below.

### Formation of the channels 26'

One or more channel(s) 26' along the substantially absorbent material free area(s) 26 in the absorbent core 28 may start forming when the absorbent material 60 absorbs a liquid and starts swelling. As the absorbent core 28 absorbs more liquid, the depressions within the absorbent core 28 formed by the channel(s) 26' will become deeper and more apparent to the eye and the touch. The formation of the channel(s) 26' may also serve to indicate that the absorbent article 20 has been loaded with liquid bodily exudates. The core wrap bond(s) 27 should remain substantially intact at least during a first phase as the absorbent material 60 absorbs a moderate quantity of liquid bodily exudates.

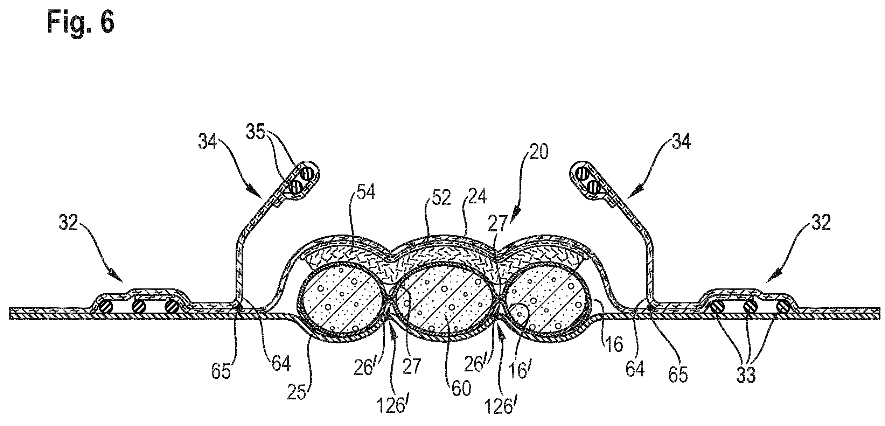

As shown in Fig. 6, when the absorbent material swells, the core wrap bonds 27 remain at least initially attached in the substantially absorbent material free areas 26. The absorbent material 60 swells in the rest of the absorbent core 28 when it absorbs a liquid, so that the core wrap thus forms channels 26' along the substantially absorbent material free areas 26 comprising the core wrap bonds 27. In that case, the colored areas 126 are still visible from the exterior of the absorbent article 20, i.e. through the backsheet 25.

### Opening of the channels 26'

In a second phase the core wrap bond(s) 27 in the channel(s) 26' can start opening to provide more space for the absorbent material 60 to swell while keeping most of the benefits of the channel(s) 26' such as increased flexibility of the absorbent core 28 in transversal direction and liquid bodily exudate management. The colored area(s) 126' at the channel(s) 26' start(s) becoming less visible from the exterior of the absorbent article 20. Consequently, the color of the top side 16 of the core wrap at one or more substantially absorbent material free area(s) 26 also becomes less visible from the exterior of the absorbent article 20.

The strength of the core wrap bond(s) 27 in the channel(s) 26' may be lower than the strength of the bonds to attach a single substrate together or two or more substrates together to form the core wrap. Hence, the channel(s) 26' open without any tearing of the core wrap when the absorbent material 60 swells.

When the absorbent material 60 swells, the caregiver will see from the exterior of the absorbent article 20 that the colored area(s) 126' start(s) disappearing. The absorbent colored area(s) 126' is/are colored due to the colored top side 16 of the core wrap and the attachment of the portion of the top side 16 of the core wrap to the portion of the bottom side 16' of the core wrap by one or more core wrap bond(s) 27 which has/have started to open in the one or more channels 26'.

Also, the size of the colored area(s) 126' when the absorbent material 60 swells is smaller than the size of the colored area(s) 126 visible before the absorbent article has been loaded by any liquid bodily exudates because the core wrap bond(s) 27 start(s) opening in the substantially absorbent material free area(s) 26.

The colored area(s) 126' is/are thus visible dryness indicia(s) indicating that the absorbent article 20 is not yet fully loaded since the disappearance of the colored area(s) 126' happen(s) when the core wrap bond(s) 27 open(s). Hence, the disappearing colored area(s) 126' indicate(s) the presence of a liquid bodily exudate in the absorbent core 28.

Fig. 8B is an outside perspective view of a front and a side of a fastenable absorbent article 20 when it is worn by a user and after the absorbent article 20 has been loaded by liquid bodily exudates. The core wrap bonds 27 start opening in order to provide more space for the absorbent material 60 to swell. In Fig. 8B, the size of the channels 26' decrease compared to the one in Fig. 8A. The colored areas 126' as shown become less and less visible from the exterior of the absorbent article 20 (i.e. through the backsheet 25). Because the colored areas 126' become less and less visible, the caregiver gets the information that the absorbent article 20 is no longer dry, i.e. the absorbent article 20 has been loaded with liquid bodily exudates.

The strength of the core wrap bond 27 within the channel 26' can be controlled for example by varying the amount and nature of the adhesive used for the attaching the two sides of the core wrap, the pressure used to make the core wrap bond and/or the distribution of the absorbent material 60, as more absorbent material 60 will usually causes more swelling and will put more pressure on the bond. The extensibility of the material of the core wrap may also play a role.

The core wrap bond 27 along a substantially absorbent material free area 26 has a bond strength that may vary from the crotch region 81 to the front and back region 82, 83 of the absorbent core 28. The variation of the bond strength of the core wrap bond 27 may be due to several factors such as the geometry of the substantially absorbent material free area 26, the amount of the absorbent material 60 in the absorbent core 28, the pattern of the adhesive, etc.

Advantageously, the bond strength of the core wrap bond 27 may be higher at the crotch region 81 than at front and back region 82, 83 of the absorbent core 28. Indeed, when the absorbent material 60 swells, the core wrap bond 27 opens from the front and back region 82, 83 to the crotch region 81 of the absorbent core 28.

Alternatively, the bond strength of the core wrap bond 27 may be higher at the front and back region 82, 83 than at the crotch region 81 of the absorbent core 28. Indeed, when the absorbent material 60 swells, the core wrap bond 27 opens from the crotch region 81 to the front and back region 82, 83 of the absorbent core 28.

The caregiver can thus see a progressive disappearance of the colored area(s) 126' visible from the exterior of the absorbent article 20, in relation of the progressing loading of the absorbent core 28. The fullness of the absorbent article 20 can be therefore determined by correlating a change in visual state of the colored area(s) 126' visible from the exterior of the absorbent article 20 to the current degree of loading of the absorbent article 20. The colored area(s) 126' can thus indicate the degree to which a liquid bodily exudate has filled the absorbent article 20.

In that respect, one may consider marking the colored area(s) 126, 126' visible from the exterior of the absorbent article 20 in the form of any symbol(s), text(s) to indicate how the absorbent core 28 is loaded. One may also consider marking the backsheet 25 at certain positions of the colored areas (126, 126') by any symbol(s) to help the caregiver to follow the progressive disappearing of the colored areas 126, 126'. For instance, a mark "M" for medium may be located between the front or back region 82, 83 and the crotch region 81 of the absorbent core 28.

The resistance to delamination of the core wrap bond in the channel(s) 26' can be quantified using the Channel Delamination Dunk Test described further below, which in short involves plunging the absorbent core in an excess of saline solution at 0.9% by weight at 37°C and recording the amount of channels that delaminate. Although not limiting the scope of first aspect of the invention, the behavior of core wrap bonds may be such that:
- less than one third of the core wrap bond's initial length delaminates at 10 min;
- from one third to two thirds of the core wrap bond's initial length delaminates at 20 min;
- at least two thirds of the core wrap bond's initial length delaminates at 60 min,
- without any delamination of the front and back end seals of the absorbent core 28 or any tearing of any substrates of the core wrap.

The channels 26' are three dimensional and can thus serve to distribute an insulting liquid bodily exudate along their length to a wider area of the absorbent core 28. This may provide a quicker liquid bodily exudate acquisition speed and a better utilization of the absorbent capacity of the absorbent core 28.

### Complete opening of the channels 26'

In a third phase, corresponding to a very high saturation of the absorbent core 28, the core wrap bond(s) 27 in the channel(s) 26' can open further, optionally completely, to provide even more space for the swelling absorbent material 60 to expand. The color of the top side 16 of the core wrap at one or more substantially absorbent material free area(s) 26 is no longer visible from the exterior of the absorbent article 20. Hence, the colored area(s) 126 initially visible from the exterior of the absorbent article 20 and then the colored area(s) 126' still visible from the exterior of the absorbent article 20 when the core wrap bond(s) 27 start(s) opening have also completely disappeared from the backsheet 25. Therefore, the absorbent article 20 is fully loaded, i.e. the absorbent article 20 has reached its full capacity. Hence, the colored area(s) 126, 126' visible from the exterior of the absorbent article 20 may provide visual fullness indicias.

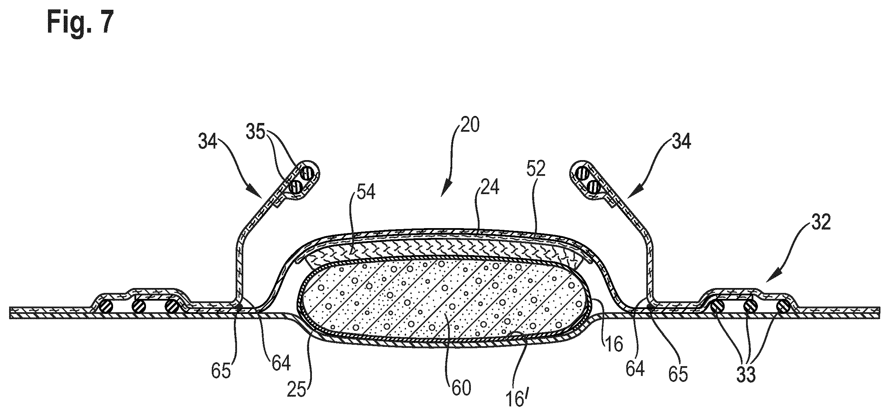

As shown in Fig. 7, when the absorbent material 60 further swells until the absorbent core 28 is fully loaded, the core wraps bonds 27 may completely open. The channels 26' have thus disappeared. Hence, the colored areas 126, 126' visible from the exterior of the absorbent article 20 have also completely disappeared from the backsheet 25.

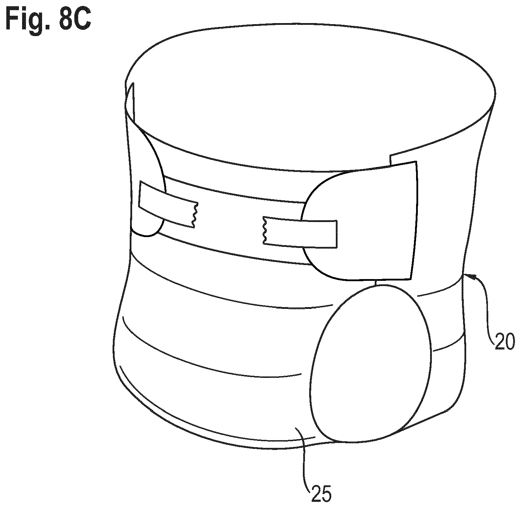

Fig. 8C is an outside perspective view of a front and a side of a fastenable absorbent article 20 when it is worn by a user and after the absorbent article 20 has been fully loaded by liquid bodily exudates. The core wrap bonds 27 are completely opened. The channels 26' as the colored areas 126, 126' visible from the exterior of the absorbent article 20 have completely disappeared. The visual dryness indicias (the colored areas 126, 126') are completely gone, which indicates that the absorbent article 20 has reached its full capacity.

### Relations between the layers and components

Typically, adjacent layers will be joined together using conventional bonding method such as adhesive coating via adhesive slot coating or spraying on the whole or part of the surface of the layer, or thermo-bonding, or pressure bonding or combinations thereof. This bonding is not represented in the Figures (except for the bonding 65 between the raised element of the leg cuffs 34 with the topsheet 24) for clarity and readability but bonding between the layers of the absorbent article 20 should be considered to be present unless specifically excluded. Adhesives may be typically used to improve the adhesion of the different layers, for example between the backsheet 25 and the core wrap. The adhesive may be any standard hotmelt adhesive as known in the art.

### Experimental settings

The values indicated herein are measured according to the methods indicated herein below, unless specified otherwise. All measurements are performed at 21°C ± 2°C and 50% ± 20% RH, unless specified otherwise. All samples should be kept at least 24 hours in these conditions to equilibrate before conducting the tests, unless indicated otherwise. All measurements should be reproduced on at least 4 samples and the average value obtained indicated, unless otherwise indicated.

### Centrifuge Retention Capacity (CRC)

The CRC measures the liquid absorbed by the superabsorbent polymer particles for free swelling in excess liquid. The CRC is measured according to EDANA method WSP 241.2-05.

### Dry Absorbent Core Caliper Test

This test may be used to measure the caliper of the absorbent core (before use i.e. without liquid bodily exudate loading) in a standardized manner.

Equipment: Mitutoyo manual caliper gauge with a resolution of 0.01 mm -- or equivalent instrument.

Contact Foot: Flat circular foot with a diameter of 17.0 mm (± 0.2 mm). A circular weight is applied to the foot (e.g., a weight with a slot to facilitate application around the instrument shaft) to achieve the target weight. The total weight of foot and added weight (including shaft) is selected to provide 2.07 kPa (0.30 psi) of pressure to the sample.

The caliper gauge is mounted with the lower surface of the contact foot in an horizontal plane so that the lower surface of the contact foot contacts the center of the flat horizontal upper surface of a base plate approximately 20 x 25 cm. The gauge is set to read zero with the contact foot resting on the base plate.
Ruler: Calibrated metal ruler graduated in mm.
Stopwatch: Accuracy 1 second
Sample preparation: The absorbent core is conditioned at least 24 hours as indicated above.

Measurement procedure: The absorbent core is laid flat with the bottom side, i.e. the side intended to be placed towards the backsheet in the finished absorbent article facing down. The point of measurement, which is the intersection of the longitudinal axis 80' and the transversal axis 90' of the absorbent core (the crotch point C) is carefully drawn on the top side of the absorbent core taking care not to compress or deform the absorbent core.

The contact foot of the caliper gauge is raised and the absorbent core is placed flat on the base plate of the caliper gauge with the top side of the absorbent core up so that when lowered, the center of the foot is on the marked measuring point.

The foot is gently lowered onto the article and released (ensure calibration to "0" prior to the start of the measurement). The caliper value is read to the nearest 0.01 mm, 10 seconds after the foot is released.

The procedure is repeated for each measuring point. If there is a fold at the measuring point, the measurement is done in the closest area to this point but without any folds. Ten articles are measured in this manner for a given product and the average caliper is calculated and reported with an accuracy of one tenth mm.

### Absorbent Article Caliper Test

The Absorbent Article Caliper Test can be performed as for the Dry Absorbent Core Caliper Test with the difference that the caliper of the finished absorbent article is measured instead of the caliper of the absorbent core. The point of measurement is the intersection of the longitudinal axis 80 and transversal axis 90 of the absorbent article. If the absorbent articles were provided folded and/or in a package, the absorbent articles to be measured are unfolded and/or removed from the center area of the package. If the package contains more than 4 absorbent articles, the outer most two absorbent articles on each side of the package are not used in the testing. If the package contains more than 4 but fewer than 14 absorbent articles, then more than one package of articles is required to complete the testing. If the package contains 14 or more absorbent articles, then only one package of absorbent articles is required to perform the testing. If the package contains 4 or fewer absorbent articles then all absorbent articles in the package are measured and multiple packages are required to perform the measurement. Caliper readings should be taken 24 ± 1 hours after the absorbent article is removed from the package, unfolded and conditioned. Physical manipulation of product should be minimal and restricted only to necessary sample preparation.

Any elastic components of the absorbent article that prevent the absorbent article from being laid flat under the caliper foot are cut or removed. These may include leg cuffs or waistbands. Pant-type articles are opened or cut along the side seams as necessary. Apply sufficient tension to flatten out any folds/wrinkles. Care is taken to avoid touching and/or compressing the area of measurement.

### Channel Delamination Dunk Test

The Test is conducted on an absorbent core to determine the behavior of the core wrap bond when submitted to an excess of saline fluid as a function of time. The Test is preferably conducted on an isolated absorbent core before it is built in a finished article.

The absorbent core may also be extracted from a finished absorbent article. In that case freeze-spray may be used to facilitate removal of the absorbent core if necessary. Care is taken to avoid damaging the absorbent core e.g. tearing the core wrapping layers. Any cuffs, fastening members, topsheet, backsheet, or absorbent layers above and/or below the absorbent core are disposed of.

If any layers are strongly attached to the laminate absorbent core such that they only be partly removed, those layers should be removed to the extent possible without damaging or destroying the laminate absorbent core. If a layer cannot be removed at all due to strong attachment to the laminate absorbent core, this layer will remain on the laminate absorbent core for the further test procedure.

The core wrap bond(s), which typically follow(s) and approximate(s) the substantially absorbent material free area(s), are marked on the body-facing surface of the core wrap with a permanent felt-tipped marker and the curvilinear length of the areas is measured. If the bonds are intermittent, the full length is marked spanning the gaps between the bonds. The absorbent core is placed in a suitable flat-bottomed container with the body-facing surface up. The container is large enough to allow the absorbent core to lie completely flat, and is about 6 cm deep. The container with the absorbent core is placed in an incubator or other suitable temperature-controlled chamber at 37 ± 0.5°C and allowed to reach thermal equilibrium. A 0.90 w.% solution of sodium chloride is prepared by dissolving the appropriate amount of pure sodium chloride in distilled water, and the solution is heated to 37°C. The heated solution is added rapidly to the container to a depth of about 4 cm and a timer is started upon addition of the fluid. Once the fluid is added, the container is immediately covered with cling film (e.g. Saran Wrap^{®}) to prevent excessive evaporation from the container, and the system is maintained at 37 ± 0.5°C.

The absorbent core is observed through the cling wrap without disturbing the system at 5 minute intervals, and the extent of delamination of any channels is recorded along with the elapsed time. The extent of delamination is the total length of any portion or portions of the channel(s) where the top side of the core wrap is no longer attached to the bottom side of the core wrap in the channel area. The fractional delaminated length is the total delaminated channel length divided by the total initial channel length prior to fluid addition. The percent delaminated channel length is the fractional delaminated channel length times 100.

### EXPERIMENTALS

The following absorbent core and absorbent article according to the invention was prepared:

### Invention example 1:

The absorbent cores tested in this example were similar to the absorbent core illustrated in Fig. 3A. The absorbent cores contained SAP as absorbent material, without cellulosic fibers. The core wrap comprised two substrates forming the top and bottom sides of the absorbent core, the first substrate of the core wrap facing the upper side of the absorbent core and forming a C-wrap along the longitudinal edges of the absorbent core and the front and back edges of the absorbent core being attached flat. The absorbent core comprised two substantially absorbent material free areas 26 in the crotch region. The substantially absorbent material free areas 26 free of SAP were symmetric in relation to the longitudinal axis 80 had a projected length thereon of about 227 mm, a width of about 8 mm and a shortest distance from each other of 20 mm. The core wrap was further attached to itself through the channels.

The absorbent core comprised in total 14.1 g fast absorbing SAP applied in an absorbent material deposition area having a length of 360mm and a width of 110 mm (rectangular profile). The SAP was distributed so that the basis weight of SAP was higher in the crotch region than at the front region and still lower towards the back region. There was no profiling of the SAP in the transversal direction ("cross-machine direction" or "CD", except of the channels which were free of absorbent material). The absorbent core was formed by SAP printing technology, as disclosed in US2010/0051166A1, which combines two nonwoven substrates each supporting a SAP layer and having a microfiber elastic adhesive applied on each SAP layer which immobilizes the SAP layer on the substrate. The substantially absorbent material free areas 26 were formed by using a suitable printing drum delimiting the channels shape, further information on how to form channels can be found in EP application number EP12174117.7 using printed SAP technology.

An adhesive was applied between the SAP layer and the first substrate of the core wrap, and was slot coated with 41 slots 1 mm wide with a distance of 1 mm between the slots along the whole length of the core wrap (390 mm). 0.211 g and 0.168 g of microfiber adhesive (from H. B. Fuller) were respectively applied on the upper and lower SAP layer, the area of application having a width of 110 mm and length of 390 mm on each SAP layer.

The core wrap had a length of 390 mm with two end flaps free of absorbent material having a length of 15 mm at the back and at the front edge of the absorbent core. The front and back end seals of the absorbent core were slot glued together, the adhesive lines having a length of 30mm from the front end seal and 20mm from the back end seal. The folded width of the core wrap was 120 mm.

The top side 16 of the core wrap was made of a first substrate which was a 10 gsm hydrophilically treated SMMS nonwoven and the bottom side 16' of the core wrap was made of a second substrate which was a 10 gsm SMMS nonwoven. The first substrate of the core wrap was cut at a length of 390 mm and a cut width of 165 mm. The first substrate of the core wrap was entirely colored by pigmentation using a Pantone color 270 sanylen violet PP 42000634 ex Clariant. The second substrate of the core wrap had a cut length of 390 mm and a cut width of 130 mm. The first substrate of the core wrap was C-wrapped around the second substrate of the core wrap on the transversal edges of the absorbent core and the transversal edges of the lower layer was slightly formed upwards on the edge of the absorbent material of the absorbent core so that the overall width of the folded core wrap was about 120 mm. The C-wrap was made permanent by application between the substrates of a core folding adhesive applied at 20 gsm with 2 slots having a slot width of 3 mm and 390 mm long on each side of the absorbent core.

The first and second substrates of the core wrap were additionally bonded together through the substantially absorbent material free areas by the core wrap bonds. The core wrap bonds were formed by applying pressure and the adhesive.

The above prepared absorbent core 28 was used to prepare an absorbent article 20.

The colored areas 126 were visible through the bottom side 16' of the core wrap and through the backsheet 25.

Channels 26' along the substantially absorbent material free areas 26 in the absorbent core 28 started forming when the absorbent material started swelling.

In a second phase the core wrap bonds 27 in the channels 26' started delaminating. The channels 26' started becoming less visible from the exterior of the absorbent article 20. Consequently, the color of the top side 16 of the core wrap at the substantially absorbent material free areas 26 became less visible from the exterior of the absorbent article 20.

In a third phase, corresponding to a very high saturation of the absorbent core 28, a more substantial part of the core wrap bonds 27 in the channels 26' delaminated, until completely. The colored areas 126' visible from the exterior of the absorbent article 20 have disappeared. The color of the top side 16 of the core wrap at the substantially absorbent material free areas 26 was no longer visible from the exterior of the absorbent article 20.

### TEST RESULTS

For the absorbent core described above, two specimens (X and Y) were tested according to the Channel Delamination Dunk Test to determine the percentage of the core wrap's bond that delaminates as a function of time (in 5 mn increments). The amount of opening was reported as follows:
A when less than one third of the core wrap bond's initial length delaminated;
B when from one third to two thirds of the core wrap bond's initial length delaminated;
C when at least two thirds of the core wrap bond's initial length delaminated.

| | % Delaminated Channel Length | |
|---|---|---|
| Sample | 1 | |
| Specimen → Time (min) ↓ | X1 | Y1 |
| 5 | A | A |
| 10 | A | A |
| 15 | A | A |
| 20 | A | A |
| 25 | A | B |
| 30 | B | B |
| 35 | B | B |
| 40 | B | B |
| 45 | C | C |
| 50 | C | C |
| 55 | C | C |
| 60 | C | C |

In all the case, the core wrap bonds delaminate before any tearing of the one or more substrates making the core wrap or before any opening of the front and back end seal of the absorbent core 28.

## Claims

1. An absorbent article (20) for personal hygiene comprising:
a liquid permeable topsheet (24),
a liquid impermeable backsheet (25),
an absorbent core (28) between the topsheet and backsheet, the absorbent core (28) comprising a core wrap enclosing an absorbent material (60), the absorbent material (60) comprising a superabsorbent polymer,
wherein the core wrap comprises a top side (16) facing the topsheet (24) and a bottom side (16') facing the backsheet (25),
wherein the absorbent core (28) comprises one or more substantially absorbent material free area(s) (26) through which a portion of the top side (16) of the core wrap is attached by one or more core wrap bond(s) (27) to a portion of the bottom side (16') of the core wrap,
**characterized in that**
the top side (16) of the core wrap is at least colored in the portion of the top side (16) of the core wrap which is attached by one or more core wrap bond(s) (27) to the portion of the bottom side (16') of the core wrap so that the color of the top side (16) of the core wrap forms one or more colored area(s) (126) visible from the exterior of the absorbent article (20), and
when the absorbent material (60) swells, one or more core wrap bond(s) (27) at least partially open(s) so that the colored area(s) (126) becomes no longer or less visible from the exterior of the absorbent article (20).

2. The absorbent article (20) according to claim 1 wherein when the absorbent material swells, the core wrap forms one or more channel(s) (26') along the substantially absorbent material free area(s) (26).

3. The absorbent article (20) according to any of the preceding claims wherein the top side 16 of the core wrap is made of a pigmented nonwoven substrate.

4. The absorbent article (20) according to claims 1 or 2 wherein the top side (16) of the core wrap includes a colored area (161) which encompasses the one or more substantially absorbent material free area(s) (26) and which is larger than the one or more substantially absorbent material free area(s) (26) but smaller that the whole top side (16) of the core wrap.

5. The absorbent article (20) according to claims 1 or 2 wherein the top side (16) of the core wrap includes a colored area corresponding to each substantially absorbent material free area (26).

6. The absorbent article (20) according to claim 1, 2, 4 or 5 wherein the color is imparted to the top side (16) of the core wrap by inkjet printing.

7. The absorbent article (20) according to any of the preceding claims wherein the portion of the top side (16) of the core wrap is attached to the portion of the bottom side (16') of the core wrap at the one or more substantially absorbent material free area(s) 26 by an adhesive comprising a plurality of adhesive lines (127) substantially parallel to a longitudinal axis (80') of the absorbent core (28).

8. The absorbent article (20) according to any of the preceding claims wherein the absorbent core (28) has a front region (82), a back region (83) and a crotch region (81), wherein the bond strength of the one or more core wrap bond(s) (27) is higher at the crotch region (81) than at the front and back region (82, 83) of the absorbent core (28), so that when the absorbent material (60) swells, the one or more core wrap bond(s) (27) open(s) from the front and back region (82, 83) to the crotch region (81) of the absorbent core (28).

9. The absorbent article (20) according to claims 1-7 wherein the bond strength of the one or more core wrap bond(s) (27) is higher at the front and back region (82, 83) than at the crotch region (81) of the absorbent core (28), so that when the absorbent material (60) swells, the one or more core wrap bond(s) (27) open(s) from the crotch region (81) to the front and back region (82, 83) of the absorbent core (28).

10. The absorbent article (20) according to any of the preceding claims, wherein the absorbent material (60) comprises at least 80% of superabsorbent polymers, up to substantially 100% of superabsorbent polymers, by total weight of the absorbent material (60), preferably wherein the absorbent core comprises from 5 g to 60 g of superabsorbent polymers, in particular from 10 g to 50 g of superabsorbent polymers.

11. The absorbent article (20) according to any of the preceding claims, wherein at least 2 substantially absorbent material free area(s) (26) are symmetrically disposed relative to the longitudinal axis of the absorbent core (80').

12. The absorbent article (20) according to any of the preceding claims, wherein one or more substantially absorbent material free area(s) (26) have a length L' projected on the longitudinal axis (80') of the absorbent core (28) which is at least 10% of the length L of the absorbent core (28).

13. The absorbent article (20) according to any of the preceding claims, wherein one or more substantially absorbent material free area(s) (26) have a width (Wc) along at least part of its length which is at least 2 mm, in particular from 4 to 16 mm.

14. The absorbent article (20) according to any of the preceding claims wherein the backsheet (25) is bonded to the bottom side (16') of the core wrap, preferably at one or more absorbent material substantially free area(s) (26).

15. An absorbent core (28) comprising:
a core wrap enclosing an absorbent material (60),
the absorbent material (60) comprising a superabsorbent polymer,
wherein the core wrap comprises a top side (16) and bottom side (16'),
wherein the absorbent core (28) comprises one or more substantially absorbent material free area(s) (26) through which a portion of the top side (16) of the core wrap is attached by one or more core wrap bond(s) (27) to a portion of the bottom side (16') of the core wrap,
**characterized in that**
the top side (16) of the core wrap is at least colored in the portion of the top side (16) of the core wrap which is attached by one or more core wrap bond(s) (27) to the portion of the bottom side (16') of the core wrap so that the color of the top side (16) of the core wrap forms one or more colored area(s) (126) visible from the bottom side (16') of the core wrap, and
when the absorbent material (60) swells, one or more core wrap bond(s) (27) at least partially open so that the colored area(s) (126) becomes no longer or less visible from the bottom side (16') of the core wrap.

## Patentansprüche

1. Absorptionsartikel (20) zur persönlichen Hygiene, wobei der Artikel Folgendes umfasst:
eine flüssigkeitsdurchlässige Oberschicht (24);
eine flüssigkeitsundurchlässige Unterschicht (25);
einen zwischen der Oberschicht und der Unterschicht angeordneten Absorptionskern (28), wobei der Absorptionskern (28) eine Kernumwicklung umfasst, die einen Absorptionsstoff (60) einschließt, wobei der Absorptionsstoff (60) ein Superabsorber-Polymer umfasst,
wobei die Kernumwicklung eine der Oberschicht (24) zugewandte Oberseite (16) und eine der Unterschicht (25) zugewandte Unterseite (16') umfasst,
wobei der Absorptionskern (28) einen oder mehrere der im Wesentlichen absorptionsstofffreie(n) Bereich(e) (26) umfasst, durch den bzw. die ein Abschnitt der Oberseite (16) der Kernumwicklung durch eine oder mehrere Kernumwicklungsbindung(en) (27) an einen Abschnitt der Unterseite (16') der Kernumwicklung angebracht ist,
**dadurch gekennzeichnet, dass**
die Oberseite (16) der Kernumwicklung wenigstens in dem Abschnitt der Oberseite (16) der Kernumwicklung, der durch eine oder mehrere Kernumwicklungsbindung(en) (27) an dem Abschnitt der Unterseite (16') der Kernumwicklung angebracht ist, farbig ist, so dass die Farbe der Oberseite (16) der Kernumwicklung einen oder mehrere farbige(n) Bereich(e) (126) bildet, der bzw. die von der Außenseite des Absorptionsartikels (20) aus sichtbar sind, und
sich bei Anschwellen des Absorptionsstoffes (60) eine oder mehrere Kernumwicklungsbindung(en) (27) wenigstens teilweise öffnet bzw. öffnen, so dass der bzw. die farbige(n) Bereich(e) (126) von der Außenseite (20) des Absorptionsartikels aus nicht mehr oder weniger gut sichtbar sind.

2. Absorptionsartikel (20) nach Anspruch 1, wobei bei Anschwellen des Absorptionsstoffes die Kernumwicklung einen Kanal oder mehrere Kanäle (26') entlang des bzw. der im Wesentlichen absorptionsstofffreien Bereichs bzw. Bereiche (26) bildet.

3. Absorptionsartikel (20) nach einem der vorstehenden Ansprüche, wobei die Oberseite 16 der Kernumwicklung aus einem pigmentierten Vliessubstrat hergestellt ist.

4. Absorptionsartikel (20) nach Anspruch 1 oder 2, wobei die Oberseite (16) der Kernumwicklung einen farbigen Bereich (161) aufweist, der den einen bzw. die mehreren im Wesentlichen absorptionsstofffreien Bereich(e) (26) umschließt und größer ist als der eine oder die mehreren im Wesentlichen absorptionsstofffreie(n) Bereich(e) (26), jedoch kleiner als die gesamte Oberseite (16) der Kernumwicklung.

5. Absorptionsartikel (20) nach Anspruch 1 oder 2, wobei die Oberseite (16) der Kernumwicklung einen farbigen Bereich einschließt, der jedem im Wesentlichen absorptionsstofffreien Bereich (26) entspricht.

6. Absorptionsartikel (20) nach Anspruch 1, 2, 4 oder 5, wobei die Farbe auf die Oberseite (16) der Kernumwicklung durch Tintenstrahldrucken aufgetragen wird.

7. Absorptionsartikel (20) nach einem der vorstehenden Ansprüche, wobei der Abschnitt der Oberseite (16) der Kernumwicklung an dem Abschnitt der Unterseite (16') der Kernumwicklung an den einen oder die mehreren im Wesentlichen absorptionsstofffreie(n) Bereich(e) 26 durch einen Klebstoff angebracht wird, der eine Vielzahl von Klebstofflinien (127) umfasst, die im Wesentlichen parallel zu einer Längsachse (80') des Absorptionskerns (28) verlaufen.

8. Absorptionsartikel (20) nach einem der vorstehenden Ansprüche, wobei der Absorptionskern (28) einen vorderen Bereich (82), einen hinteren Bereich (83) und einen Schrittbereich (81) aufweist, wobei die Bindungskraft der einen oder der mehreren Kernumwicklungsbindung(en) (27) im Schrittbereich (81) größer ist als im vorderen bzw. hinteren Bereich (82, 83) des Absorptionskerns (28), so dass bei Anschwellen des Absorptionsstoffes (60) der eine oder die mehreren Kernumwicklungsbindung(en) (27) sich vom vorderen und hinteren Bereich (82, 83) bis zum Schrittbereich (81) des Absorptionskerns (28) öffnet bzw. öffnen.

9. Absorptionsartikel (20) nach den Ansprüchen 1 bis 7, wobei die Bindungskraft der einen oder der mehreren Kernumwicklungsbindung(en) (27) im vorderen bzw. hinteren Bereich (82, 83) größer ist als im Schrittbereich (81) des Absorptionskerns (28), so dass bei Anschwellen des Absorptionsstoffes (60) der eine oder die mehreren Kernumwicklungsbindung(en) (27) sich vom Schrittbereich (81) bis zum vorderen und hinteren Bereich (82, 83) des Absorptionskerns (28) öffnet bzw. öffnen.

10. Absorptionsartikel (20) nach einem der vorstehenden Ansprüche, wobei das Absorptionsmaterial (60) zu mindestens 80 % Superabsorber-Polymere umfasst, bis zu im Wesentlichen 100 % Superabsorber-Polymere nach dem Gesamtgewicht des Absorptionsstoffes (60), wobei vorzugsweise der Absorptionskern zu 5 g bis 60 g Superabsorber-Polymere umfasst, insbesondere zu 10 g bis 50 g Superabsorber-Polymere.

11. Absorptionsartikel (20) nach einem der vorstehenden Ansprüche, wobei mindestens 2 im Wesentlichen absorptionsstofffreie Bereiche (26) in Bezug auf die Längsachse des Absorptionskerns (80') symmetrisch angeordnet sind.

12. Absorptionsartikel (20) nach einem der vorstehenden Ansprüche, wobei ein oder mehrere im Wesentlichen absorptionsstofffreie(r) Bereich(e) (26) eine auf die Längsachse (80') des Absorptionskerns (28) projizierte Länge L', die mindestens 10 % der Länge L des Absorptionskerns (28) beträgt, aufweist bzw. aufweisen.

13. Absorptionsartikel (20) nach einem der vorstehenden Ansprüche, wobei ein oder mehrere im Wesentlichen absorptionsstofffreie(r) Bereich(e) (26) eine Breite (Wc) entlang zumindest eines Teils seiner Länge aufweist, die mindestens 2 mm beträgt, insbesondere 4 bis 16 mm.

14. Absorptionsartikel (20) nach einem der vorstehenden Ansprüche, wobei die Unterschicht (25) mit der Unterseite (16') der Kernumwicklung verbunden ist, vorzugsweise an einem oder mehreren im Wesentlichen absorptionsstofffreien Bereich(en) (26).

15. Absorptionskern (28), der Folgendes umfasst:
eine Kernumwicklung, die einen Absorptionsstoff (60) einschließt,
der Absorptionsstoff (60), der ein Superabsorber-Polymer umfasst,
wobei die Kernumwicklung eine Oberseite (16) und eine Unterseite (16') umfasst,
wobei der Absorptionskern (28) einen oder mehrere im Wesentlichen absorptionsstofffreie(n) Bereich(e) (26) umfasst, durch den bzw. die ein Abschnitt der Oberseite (16) der Kernumwicklung durch eine oder mehrere Kernumwicklungsbindung(en) (27) an einen Abschnitt der Unterseite (16') der Kernumwicklung angebracht ist,
**dadurch gekennzeichnet, dass**
die Oberseite (16) der Kernumwicklung zumindest in dem Abschnitt der Oberseite (16) der Kernumwicklung, der durch eine oder mehrere Kernumwicklungsbindung(en) (27) an dem Abschnitt der Unterseite (16') der Kernumwicklung angebracht ist, farbig ist, so dass die Farbe der Oberseite (16) der Kernumwicklung einen oder mehrere farbige(n) Bereich(e) (126) bildet, der bzw. die von der Unterseite (16') der Kernumwicklung aus sichtbar sind, und
sich bei Anschwellen des Absorptionsstoffes (60) eine oder mehrere Kernumwicklungsbindung(en) (27) zumindest teilweise öffnet bzw. öffnen, so dass der bzw. die farbige(n) Bereich(e) (126) von der Unterseite (16') der Kernumwicklung aus nicht mehr oder weniger gut sichtbar sind.

## Revendications

1. Article absorbant (20) pour hygiène personnelle comprenant :
une feuille de dessus perméable aux liquides (24),
une feuille de fond imperméable aux liquides (25),
une âme absorbante (28) entre la feuille de dessus et la feuille de fond, l'âme absorbante (28) comprenant une enveloppe d'âme enfermant un matériau absorbant (60), le matériau absorbant (60) comprenant un polymère superabsorbant,
dans lequel l'enveloppe d'âme comprend un côté supérieur (16) faisant face à la feuille de dessus (24) et un côté inférieur (16') faisant face à la feuille de fond (25),
dans lequel l'âme absorbante (28) comprend une ou plusieurs zone(s) essentiellement sans matériau absorbant (26) à travers lesquelles une partie du côté supérieur (16) de l'enveloppe d'âme est fixée par une ou plusieurs liaison(s) d'enveloppe d'âme (27) à une partie du côté inférieur (16') de l'enveloppe d'âme,
**caractérisé en ce que**
le côté supérieur (16) de l'enveloppe d'âme est au moins coloré dans la partie du côté supérieur (16) de l'enveloppe d'âme qui est fixée par une ou plusieurs liaison(s) d'enveloppe d'âme (27) à la partie du côté inférieur (16') de l'enveloppe d'âme de sorte que la couleur du côté supérieur (16) de l'enveloppe d'âme forme une ou plusieurs zone(s) colorée(s) (126) visible(s) depuis l'extérieur de l'article absorbant (20), et
lorsque le matériau absorbant (60) gonfle, une ou plusieurs liaison(s) d'enveloppe d'âme (27) s'ouvre(nt) au moins partiellement de sorte que la ou les zone(s) colorée(s) (126) ne sont plus visibles ou deviennent moins visibles depuis l'extérieur de l'article absorbant (20).

2. Article absorbant (20) selon la revendication 1, dans lequel, lorsque le matériau absorbant gonfle, l'enveloppe d'âme forme un ou plusieurs canaux (26') le long de la ou des zone(s) essentiellement sans matériau absorbant (26).

3. Article absorbant (20) selon l'une quelconque des revendications précédentes, dans lequel le côté supérieur (16) de l'enveloppe d'âme est constitué d'un substrat non tissé pigmenté.

4. Article absorbant (20) selon la revendication 1 ou 2, dans lequel le côté supérieur (16) de l'enveloppe d'âme inclut une zone colorée (161) qui englobe la ou les zone(s) essentiellement sans matériau absorbant (26) et qui est plus grande que la ou les zone(s) essentiellement sans matériau absorbant (26), mais plus petite que la totalité du côté supérieur (16) de l'enveloppe d'âme.

5. Article absorbant (20) selon la revendication 1 ou 2, dans lequel le côté supérieur (16) de l'enveloppe d'âme inclut une zone colorée correspondant à chaque zone essentiellement sans matériau absorbant (26).

6. Article absorbant (20) selon la revendication 1, 2, 4 ou 5, dans lequel la couleur est communiquée au côté supérieur (16) de l'enveloppe d'âme par impression à jet d'encre.

7. Article absorbant (20) selon l'une quelconque des revendications précédentes, dans lequel la partie du côté supérieur (16) de l'enveloppe d'âme est fixée à la partie du côté inférieur (16') de l'enveloppe d'âme au niveau de la ou des zone(s) essentiellement sans matériau absorbant (26) par un adhésif comprenant une pluralité de lignes adhésives (127) essentiellement parallèles à un axe longitudinal (80') de l'âme absorbante (28).

8. Article absorbant (20) selon l'une quelconque des revendications précédentes, dans lequel l'âme absorbante (28) a une région antérieure (82), une région postérieure (83) et une région d'entrejambe (81), dans lequel la résistance de liaison de la ou des liaison(s) d'enveloppe d'âme (27) est plus élevée au niveau de la région d'entrejambe (81) qu'au niveau de la région antérieure et postérieure (82, 83) de l'âme absorbante (28), de sorte que, lorsque le matériau absorbant (60) gonfle, la ou les liaison(s) d'enveloppe d'âme (27) s'ouvre(nt) de la région antérieure et postérieure (82, 83) à la région d'entrejambe (81) de l'âme absorbante (28).

9. Article absorbant (20) selon les revendications 1 à 7, dans lequel la résistance de liaison de la ou des liaison(s) d'enveloppe d'âme (27) est plus élevée au niveau de la région antérieure et postérieure (82, 83) qu'au niveau de la région d'entrejambe (81) de l'âme absorbante (28), de sorte que, lorsque le matériau absorbant (60) gonfle, la ou les liaison(s) d'enveloppe d'âme (27) s'ouvre(nt) de la région d'entrejambe (81) à la région antérieure et
postérieure (82, 83) de l'âme absorbante (28).

10. Article absorbant (20) selon l'une quelconque des revendications précédentes, dans lequel le matériau absorbant (60) comprend au moins 80 % de polymères superabsorbants, jusqu'à essentiellement 100 % de polymères superabsorbants, en poids total du matériau absorbant (60), de préférence dans lequel l'âme absorbante comprend de 5 g à 60 g de polymères superabsorbants, en particulier de 10 g à 50 g de polymères superabsorbants.

11. Article absorbant (20) selon l'une quelconque des revendications précédentes, dans lequel au moins 2 zones essentiellement sans matériau absorbant (26) sont disposées symétriquement par rapport à l'axe longitudinal de l'âme absorbante (80').

12. Article absorbant (20) selon l'une quelconque des revendications précédentes, dans lequel une ou plusieurs zone(s) essentiellement sans matériau absorbant (26) a(ont) une longueur L' projetée sur l'axe longitudinal (80') de l'âme absorbante (28) qui est d'au moins 10 % de la longueur L de l'âme absorbante (28).

13. Article absorbant (20) selon l'une quelconque des revendications précédentes, dans lequel une ou plusieurs zone(s) essentiellement sans matériau absorbant (26) a(ont) une largeur (Wc) le long d'au moins une partie de sa(leur) longueur qui est d'au moins 2 mm, en particulier de 4 à 16 mm.

14. Article absorbant (20) selon l'une quelconque des revendications précédentes, dans lequel la feuille de fond (25) est liée au côté inférieur (16') de l'enveloppe d'âme, de préférence au niveau d'une ou plusieurs zone(s) essentiellement exempte(s) de matériau absorbant (26).

15. Âme absorbante (28) comprenant :
une enveloppe d'âme enfermant un matériau absorbant (60),
le matériau absorbant (60) comprenant un polymère superabsorbant,
dans laquelle l'enveloppe d'âme comprend un côté supérieur (16) et un côté inférieur (16'),
dans lequel l'âme absorbante (28) comprend une ou plusieurs zone(s) essentiellement sans matériau absorbant (26) à travers lesquelles une partie du côté supérieur (16) de l'enveloppe d'âme est fixée par une ou plusieurs liaison(s) d'enveloppe d'âme (27) à une partie du côté inférieur (16') de l'enveloppe d'âme,
**caractérisé en ce que**
le côté supérieur (16) de l'enveloppe d'âme est au moins coloré dans la partie du côté supérieur (16) de l'enveloppe d'âme qui est fixée par une ou plusieurs liaison(s) d'enveloppe d'âme (27) à la partie du côté inférieur (16') de l'enveloppe d'âme de sorte que la couleur du côté supérieur (16) de l'enveloppe d'âme forme une ou plusieurs zone(s) colorée(s) (126) visible(s) depuis le côté inférieur (16') de l'enveloppe d'âme, et
lorsque le matériau absorbant (60) gonfle, une ou plusieurs liaison(s) d'enveloppe d'âme (27) s'ouvre(nt) au moins partiellement de sorte que la ou les zone(s) colorée(s) (126) ne sont plus visibles ou deviennent moins visibles depuis le côté inférieur (16') de l'enveloppe d'âme.
